# FASCICULE DE BREVET EUROPEEN

(11) **EP 0 998 465 B1**
(45) Date de publication et mention de la délivrance du brevet: **10.09.2003**
(21) Numéro de dépôt: 98939678.3
(22) Date de dépôt: 15.07.1998
(51) Int. Cl.: C07D 241/12, A61K 31/495

(54) **DERIVES DE POLYHYDROXYLALKYLPYRAZINE, LEUR PREPARATION, MEDICAMENTS LES CONTENANT**
POLYHYDROXYALKYLPYRAZINE DERIVATE, DEREN HERSTELLUNG,DIESE ENTHALTENDE ARZNEIMITTELN
POLYHYDROXYALKYLPYRAZINE DERIVATIVES, PREPARATION AND MEDICINES CONTAINING THEM

(30) Priorité: 17.07.1997 FR 9709060
(43) Date de publication de la demande: 10.05.2000
(73) Titulaire: Aventis Pharma S.A., 92160 Antony (FR)
(72) Inventeur: BASHIARDES, Georges, F-94320 Thiais (FR); CARRY, Jean-Christophe, F-92190 Meudon (FR); EVERS, Michel, F-94510 La Queue en Brie (FR); FILOCHE, Bruno, F-94000 Créteil (FR); MIGNANI, Serge, F-92290 Châtenay-Malabry (FR)
(86) Numéro de dépôt international: FR9801544
(87) Numéro de publication internationale: WO99003842

(56) Documents cités:
- WO-A-97/28813
- CHEMICAL ABSTRACTS, vol. 73, no. 21, 1970 Columbus, Ohio, US; abstract no. 107839q, K.NISHIE: "PHARMACOL.OF ALK. AND HYDROXYALKYLPYRAZINES." page 216; XP002060214 & TOXICOL.APPL.PHARM., vol. 17, no. 1, 1970, pages 244-249, CALIF
- CHEMICAL ABSTRACTS, vol. 109, no. 22, 1988 Columbus, Ohio, US; abstract no. 169743z, R.HARDT: "CURIE-POINT PYROLYSIS" page 655; XP002060298 & J.ANAL.APPL.PYROLYSIS, vol. 13, no. 3, 1988, pages 191-198, ENGL
- CHEMICAL ABSTRACTS, vol. 92, no. 33, 1980 Columbus, Ohio, US; abstract no. 76832t, S.EITELMAN ET AL.: "DECOMPOSITION REACTION OF AMINO SUGARS" page 715; XP002060270 & CARBOHYDR.RES., vol. 77, 1979, pages 205-211, USA
- CHEMICAL ABSTRACTS, vol. 105, no. 17, 1986 Columbus, Ohio, US; abstract no. 170812v, H.TSUCHIDA: "IDENTIFICATION OF NOVEL PYRAZINES" page 607; XP002060325 & DEV.FOOD SCI., vol. 13, 1986, pages 85-94, JP

## Description

La présente invention concerne les médicaments contenant en tant que principe actif au moins un composé de formule générale : 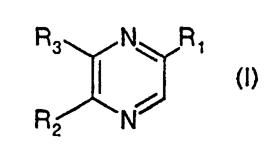 sous leurs formes stéréoisomères, leurs sels avec un acide minéral ou organique, les nouveaux composés de formule (I), leurs sels avec un acide minéral ou organique et leur préparation.

Dans la formule générale (I):
R₁ représente les formes stéréoisomères de la chaîne

   -(CHOH)₃-CH₂OH (II)

   et
soit R₂ représente un atome d'hydrogène et R₃ représente les formes stéréoisomères de la chaîne

   -CH₂-(CHOH)₂-CH₂OH (III)
soit R₂ représente les formes stéréoisomères des chaînes

   -(CHOH)₃-CH₂OH (II)

   ou

   -CH₂-(CHOH)₂-CH₂OH (III)
et R₃ représente un atome d'hydrogène
   à l'exception de
   - la fructosazine de formule 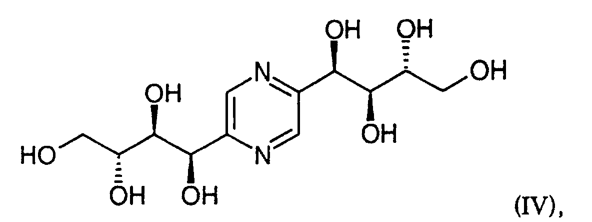
   - la déoxyfructosazine de formule 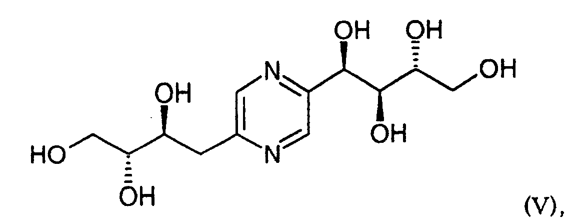
   - et du composé de formule

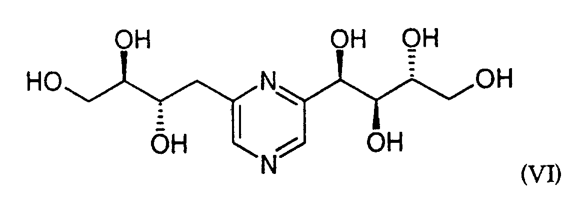

Les médicaments selon l'invention contiennent donc au moins un stéréoisomère des composés suivants : 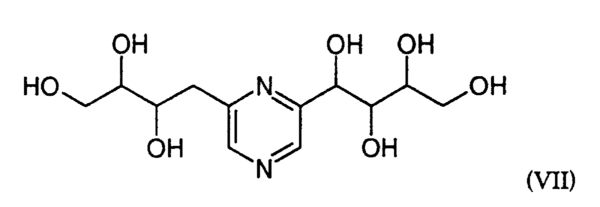 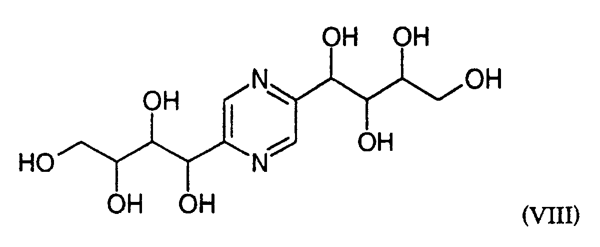 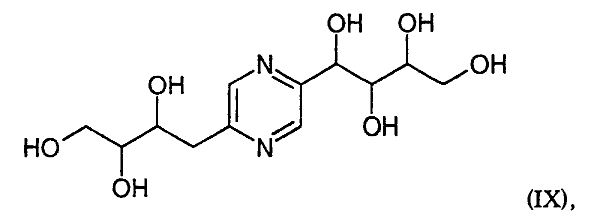 ou un sel d'un tel composé avec un acide organique ou minéral, à l'exception de la fructosazine, de la déoxyfructosazine et du composé de formule (VI).

Les médicaments selon l'invention sont de préférence ceux qui contiennent en tant que principe actif au moins un composé de formule (I) choisi parmi les composés suivants :
1-[5-(1R,2R,3R,4-tétrahydroxy-butyl)-pyrazin-2-yl]-butane-1R,2R,3R,4-tétraol
1-[5-(1R,2R,3S,4-tétrahydroxy-butyl)-pyrazin-2-yl]-butane-1R,2R,3S,4-tétraol
1-[5-(1R,2S,3S,4-tétrahydroxy-butyl)-pyrazin-2-yl]-butane-1R,2S,3S,4-tétraol
1-[5-(1S,2R,3R,4-tétrahydroxy-butyl)-pyrazin-2-yl]-butane-1S,2R,3R,4-tétraol
1-[5-(1S,2R,3S,4-tétrahydroxy-butyl)-pyrazin-2-yl]-butane-1S,2R,3S,4-tétraol
1-[5-(1S,2S,3R,4-tétrahydroxy-butyl)-pyrazin-2-yl]-butane-1S,2S,3R,4-tétraol
1-[5-(1S,2S,3S,4-tétrahydroxy-butyl)-pyrazin-2-yl]-butane-1S,2S,3S,4-tétraol
1-[5-(2R,3R,4-trihydroxy-butyl)-pyrazin-2-yl]-butane-1R,2R,3R,4-tétraol
1-[5-(2R,3S,4-trihydroxy-butyl)-pyrazin-2-yl]-butane-1R,2R,3S,4-tétraol
1-[5-(2S,3S,4-trihydroxy-butyl)-pyrazin-2-yl]-butane-1R,2S,3S,4-tétraol
1-[5-(2R,3R,4-trihydroxy-butyl)-pyrazin-2-yl]-butane-1S,2R,3R,4-tétraol
1-[5-(2R,3S,4-trihydroxy-butyl)-pyrazin-2-yl]-butane-1S,2R,3S,4-tétraol
1-[5-(2S,3R,4-trihydroxy-butyl)-pyrazin-2-yl]-butane-1S,2S,3R,4-tétraol
1-[5-(2S,3S,4-trihydroxy-butyl)-pyrazin-2-yl]-butane-1S,2S,3S,4-tétraol
1-[6-(2R,3R,4-trihydroxy-butyl)-pyrazin-2-yl]-butane-1R,2R,3R,4-tétraol
1-[6-(2R,3S,4-trihydroxy-butyl)-pyrazin-2-yl]-butane-1R,2R,3S,4-tétraol
1-[6-(2R,3R,4-trihydroxy-butyl)-pyrazin-2-yl]-butane-1S,2R,3R,4-tétraol
1-[6-(2R,3S,4-trihydroxy-butyl)-pyrazin-2-yl]-butane-1S,2R,3S,4-tétraol
1-[6-(2S,3R,4-trihydroxy-butyl)-pyrazin-2-yl]-butane-1S,2S,3R,4-tétraol
1-[6-(2S,3S,4-trihydroxy-butyl)-pyrazin-2-yl]-butane-1S,2S,3S,4-tétraol.
ou un sel d'un tel composé avec un acide minéral ou organique,
et de façon encore plus avantageuse, ceux qui contiennent en tant que principe actif au moins un composé de formule (I) choisi parmi les composés suivants :
1-[5-(1R,2R,3R,4-tétrahydroxy-butyl)-pyrazin-2-yl]-butane-1R,2R,3R,4-tétraol
1-[5-(2R,3R,4-trihydroxy-butyl)-pyrazin-2-yl]-butane-1R,2R,3R,4-tétraol
1-[5-(2S,3R,4-trihydroxy-butyl)-pyrazin-2-yl]-butane-1S,2S,3R,4-tétraol
1-[6-(2R,3S,4-trihydroxy-butyl)-pyrazin-2-yl]-butane-1S,2R,3S,4-tétraol
1-[6-(2R,3R,4-trihydroxy-butyl)-pyrazin-2-yl]-butane-1R,2R,3R,4-tétraol
ou un sel d'un tel composé avec un acide minéral ou organique.

Les composés suivants sont connus :
- la fructosazine, la déoxyfructozasine et le composé de formule (VI) sont décrits (brevet JP 43-13469, Ann., 644, 122-127 (1961); Agr. Biol. Chem, 39 (5), 1143-1148 (1975) et dans la demande internationale WO 97/28813, qui a été publiée après la date de priorité de la présente demande).
- les stéréoisomères de formule générale (VIa), (VIb), (VIc) et (VId) mentionnés ci-dessous ont été décrits (brevet JP 43-13469, Carbohyd. Res., 26(2), 377-84 (1973), J. Anal.Appl.Pyrolysis 13,191-198(1988)) 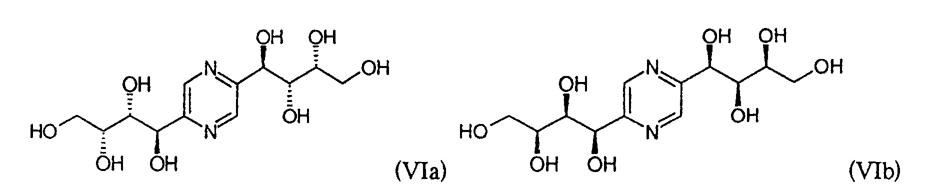 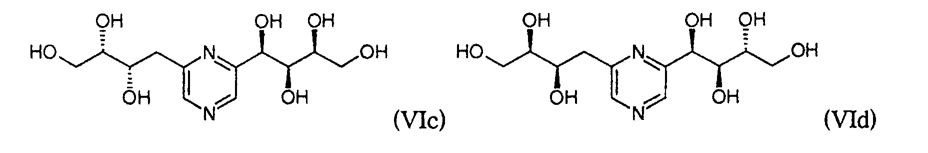
- les composés de formules générales (VII), (VIII), (IX) issus du glucose, fructose, mannose, galactose ont été décrits dans le brevet japonais JP 53-90401.

Cependant, leur utilisation en tant que médicament n'a pas été mentionnée et c'est l'objet de la présente invention.

Les composés de formule (I), ou leurs sels avec un acide minéral ou organique, à l'exception des composés suivants : 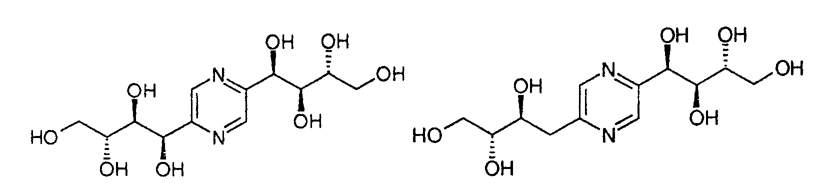 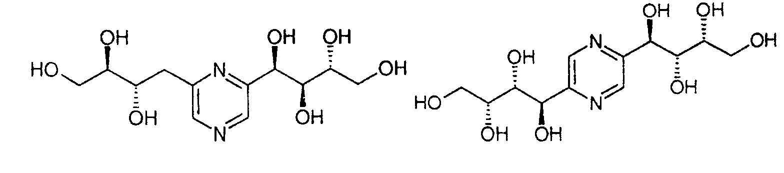 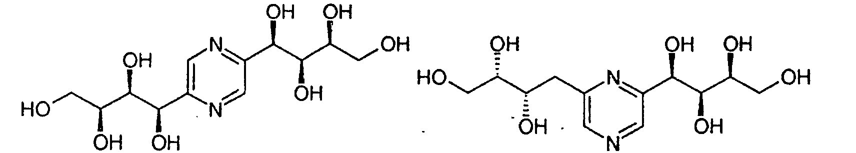 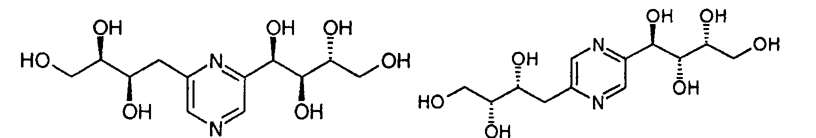 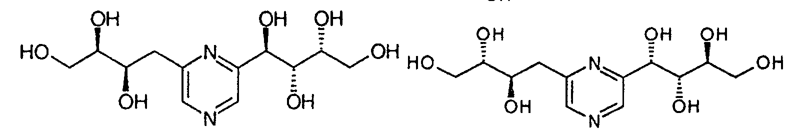 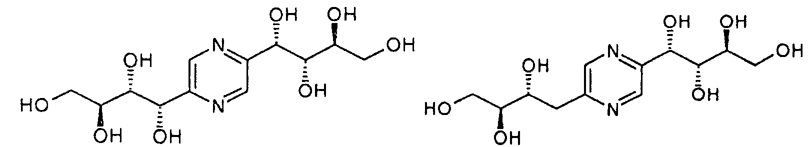 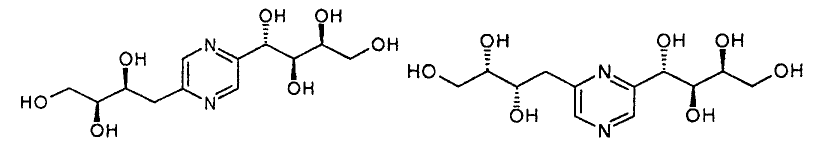 sont nouveaux et en tant que tel font partie de l'invention.

Les composés de formule (I) préférés sont les suivants :
1-[5-(1R,2R,3S,4-tétrahydroxy-butyl)-pyrazin-2-yl]-butane-1R,2R,3S,4-tétraol ; 1-[5-(1S,2R,3R,4-tétrahydroxy-butyl)-pyrazin-2-yl]-butane-1S,2R,3R,4-tétraol ; 1-[5-(1S,2S,3R,4-tétrahydroxy-butyl)-pyrazin-2-yl]-butane-1S,2S,3R,4-tétraol ; 1-[5-(1S,2S,3S,4-tétrahydroxy-butyl)-pyrazin-2-yl]-butane-1S,2S,3S,4-tétraol ; 1-[5-(2R,3S,4-trihydroxy-butyl)-pyrazin-2-yl]-butane-1R,2R,3S,4-tétraol; 1-[5-(2S,3S,4-trihydroxy-butyl)-pyrazin-2-yl]-butane-1R,2S,3S,4-tétraol ; 1-[5-(2R,3R,4-trihydroxybutyl)-pyrazin-2-yl]-butane-1S,2R,3R,4-tétraol ; 1-[5-(2S,3R,4-trihydroxy-butyl)-pyrazin-2-yl]-butane-1S,2S,3R,4-tétraol ; 1-[6-(2R,3S,4-trihydroxy-butyl)-pyrazin-2-yl]-butane-1R,2R,3S,4-tétraol ; 1-[6-(2R,3R,4-trihydroxy-butyl)-pyrazin-2-yl]-butane-1S,2R,3R,4-tétraol ; 1-[6-(2S,3R,4-trihydroxy-butyl)-pyrazin-2-yl]-butane-1S,2S,3R,4-tétraol; ou un sel d'un tel composé avec un acide minéral ou organique, avantageusement :
1-[5-(2S,3S,4-trihydroxy-butyl)-pyrazin-2-yl]-butane-1R,2S,3S,4-tétraol ; 1-[5-(2R,3R,4-trihydroxy-butyl)-pyrazin-2-yl]-butane-1S,2R,3R,4-tétraol ; 1-[5-(2S,3R,4-trihydroxy-butyl)-pyrazin-2-yl]-butane-1S,2S,3R,4-tétraol ou un sel d'un tel composé avec un acide minéral ou organique;
et de façon encore plus avantageuse le composé suivant : 1-[5-(2S,3R,4-trihydroxybutyl)-pyrazin-2-yl]-butane-1S,2S,3R,4-tétraol ou un sel d'un tel composé avec un acide minéral ou organique.

Les formes stéréoisomères des composés de formule générale (I) sont obtenus à partir des formes stéréoisomères des réactifs ci-après utilisés par le procédé de préparation selon l'invention.

Les stéréoisomères des composés de formule (I) pour lesquels R₁ représente les formes stéréoisomères de la chaîne -(CHOH)₃-CH₂OH (II), R₂ représente un atome d'hydrogène et R₃ représente les formes stéréoisomères de la chaîne -CH₂-(CHOH)₂-CH₂OH (III) c'est-à-dire les composés représentés par la formule générale (VII) peuvent être obtenus par action du formiate d'ammonium sur un aldose, ou un mélange de 2 aldoses, de série dextrogyre ou lévogyre de formule générale :

CHO-CHOH-R₁ (X)

dans laquelle R₁ a la même signification que dans la formule (I).

Cette réaction peut être effectuée préférentiellement à une température comprise entre 15°C et 100°C, de préférence en milieu aqueux.

Les aldoses sont commercialisés ou peuvent être obtenus à partir :
a) d'aldoses commercialement disponibles :
   - par des réactions d'épimérisation par application ou adaptation des méthodes décrites dans Adv. Carbohydr. Chem., 13, 63, (1958) notamment en milieu basique au moyen d'une solution aqueuse diluée de soude (0,03 à 0,05%), à une température comprise entre 20 et 40°C,
   - par des réactions d'allongement de chaîne par application ou adaptation des méthodes décrites dans « The Carbohydrates », éditeurs: W. Pigman et D. Horton, Academic Press, New-York, Volume IA, 133 (1972) et notamment en formant la cyanhydrine de l'aldose de départ (par exemple par action du cyanure de sodium en solution aqueuse, à une température comprise entre 10 et 30°C et en présence de soude, à un pH voisin de 9) puis hydrolyse de la fonction nitrile ainsi formée en acide correspondant par application ou adaptation des méthodes décrites dans Organic Synthesis volume I page 436 et volume III page 85 (par exemple à l'aide d'acide chlorhydrique ou d'acide sulfurique concentré, en solution aqueuse, à une température comprise entre 20°C et la température de reflux du milieu réactionnel), puis réduction de la fonction acide carboxylique en aldéhyde correspondant par application ou adaptation des méthodes décrites dans J. Am. Chem. Soc. 71, 122 (1949) notamment à l'aide d'un borohydrure d'un métal alcalin (le borohydrure de sodium par exemple), en solution aqueuse à une température comprise entre 20°C et la température d'ébullition du milieu réactionnel,
   - par des réactions de racourcissement de chaînes par application ou adaptation des méthodes décrites dans « The Carbohydrates», éditeurs: W. Pigman et D. Horton, Academic Press, New-York, Volume IB, 1980, page 929 ou Chem. Ber., 83, 559 (1950) et notamment en transformant la fonction aldéhyde de l'aldose en hydroxylamine correspondant par application ou adaptation des méthodes décrites dans Organic Synthesis volume II page 314 (par exemple à l'aide de chlorhydrate d'hydroxylamine, en solution aqueuse et en présence d'une base telle que le carbonate de sodium à une température comprise entre 20 et 50°C), puis action du 3,4-dinitro-fluorobenzène en présence de dioxyde de carbone et d'une base telle le d'hydrogénocarbonate de sodium en solution aqueuse et d'un alcool aliphatique (alcool isopropylique par exemple), à une température comprise entre 50 et 80°C,
b) d'alcools allyliques correspondants par application ou adaptation des méthodes décrites dans Science, 220, 949 (1983) et notamment à l'aide d'hydroperoxyde de terbutyle en présence d'un complexe de titane (IV) tel que le complexe isopropylate de titane (IV) et tartrate de dialkyle optiquement pur (le tartrate de diéthyle par exemple), suivi de l'action successive de thiophénolate de sodium, d'acide parachloroperbenzoïque dans l'anhydride acétique et d'hydrure de diisopropylaluminium

Les stéréoisomères du sucre de formule (X) peuvent être ceux des aldoses à 6 atomes de carbone; ceux utilisés de façon préférentielle sont le D-gulose, le D-galactose, le D-allose, le D-altrose, le D-idose, le D-talose, le L-glucose, le L-mannose, le L-galactose, le L-allose, le L-altrose, le L-idose, le L-talose, le L-gulose.

Les stéréoisomères des composés de formule (I) pour lesquels R₁ représente les formes stéréoisomères de la chaîne -(CHOH)₃-CH₂OH (II), R₂ représente les formes stéréoisomères des chaînes -(CHOH)₃-CH₂OH (II) et R₃ représente un atome d'hydrogène c'est-à-dire des composés représentés par la formule générale (VIII) peuvent être obtenus par traitement en milieu basique d'un amino-aldose, ou d'un mélange de 2 amino-aldoses de formule générale :

CHO-CH(NH₂)-R₁ (XI)

éventuellement sous forme de sel d'addition, tel que le chlorhydrate, dans laquelle R₁ a la même signification que dans la formule générale (I).

De préférence, on opère à une température voisine de 20°C et on utilise préférentiellement une solution d'ammoniaque et plus particulièrement une solution à 28%.

Les amino-aldoses de formule (XI) sont commercialisés ou peuvent être préparés par application ou adaptation des méthodes décrites par exemple dans :
(a) Methods Carbohydr. Chem., 7, 29 (1976) qui consistent à transformer la fonction aldéhyde de l'aldose correspondant en un groupement nitroéthylénique à l'aide du nitrométhane en milieu basique (éthylate de sodium par exemple) puis à traiter le produit obtenu successivement par une solution saturée d'ammoniaque, à une température comprise entre 20°C et 30°C, par du Ba(OH)₂ en solution aqueuse, à une température comprise entre 20°C et 30°C et enfin de l'acide sulfurique dilué (10 à 15%), à une température comprise entre 20°C et 30°C,
(b) « The Amino Sugar» , éditeur: R. W. Jeanloz, Academic Press, New-York, 1969, page 1 ou « The Carbohydrates», éditeurs: W. Pigman et D. Horton, Academic Press, New-York, Volume IB, 1980, page 664 qui consistent à transformer la fonction aldéhyde de l'aldose correspondant en un groupement imino à partir d'une amine primaire aromatique (aniline par exemple), de faire ensuite successivement réagir l'acide cyanhydrique, à une température comprise entre 0°C et 20°C et de l'hydrogène en présence de palladium dans un solvant tel qu'un éther (tétrahydrofuranne par exemple) ou un alcool aliphatique (l'éthanol ou le méthanol par exemple), à une température comprise entre 20°C et 50°C.

Les stéréoisomères de l'amino-aldose de formule (XI) peuvent être ceux des amino-aldose à 6 atomes de carbone; celui utilisé de façon préférentielle est la D-galactosamine, éventuellement sous forme de sel d'addition tel que le chlorhydrate.

Les stéréoisomères des composés de formule (I) pour lesquels R₁ représente les formes stéréoisomères de la chaîne -(CHOH)₃-CH₂OH (II), R₂ représente les formes stéréoisomères des chaînes -CH₂-(CHOH)₂-CH₂OH (III)et R₃ représente un atome d'hydrogène c'est-à-dire des composés représentés par la formule générale (IX) peuvent être obtenus
soit à partir d' un amino-aldose, ou d'un mélange de 2 aminoaldoses, de formule générale :

CHO-CH(NH₂)-R₁ (XI)

dans laquelle R₁ a la même signification que dans la formule générale (I) en milieu acide et plus particulièrement en mileu acide acétique et en opérant de préférence à une température comprise entre 15°C et 100°C.
soit à partir d'un cétose, ou d'un mélange de 2 cétoses de formule générale :

HOCH₂-CO-R₁ (XII)

dans laquelle R₁ a la même signification que dans la formule générale (I), par action du formiate d'ammonium et en opérant préférentiellement à une température comprise entre 15°C et 100°C, et de préférence en milieu aqueux.

Les cétoses de formule (XII) sont commercialisés ou peuvent être préparés par application ou adaptation des méthodes décrites par exemple dans :
a) Adv. Carbohydr. Chem., 13, 63 (1958) qui consistent à faire réagir l'aldose correspondant soit avec une base telle l'hydroxyde de calcium, la soude, la pyridine, la quinoléine, soit avec un acide tel que l'acide sulfurique en solution aqueuse ou en phase pure, à une température comprise entre 20 et 50°C
b) Tetrahedron Asymmetry, 7(8), 2185, (1996), J. Am. Chem; Soc., 118(33), 7653 (1996), J. Org. Chem., 60(13), 4294 (1995), Tetrahedron Lett., 33(36), 5157 (1992), J. Am. Chem. Soc., 113(17), 6678 (1991), Angew. Chem., 100(5), 737, (1988), J. Org. Chem., 57, 5899 (1992) qui consistent par exemple à condenser soit l'hydroxypyruvaldéhyde, la 1,3-dihydroxyacétone, le 1,3-dihydroxyacétone monophosphate ou l'acide hydroxypyruvique sur un 2-hydroxy-acétaldéhyde substitué en position 2, éventuellement optiquement pur, en présence éventuellement d'une enzyme telle qu'une transcétolase. Cette réaction s'éffectue généralement en solution aqueuse, à une température comprise entre 20 et 50°C, éventuellement en présence d'une base (la soude par exemple), de chlorure de barium, de chlorure de magnésium
ou de chlorure de zinc. Les dérivés possédant un groupement 2-hydroxy-acétaldéhyde sont commercialisés ou peuvent être préparés à partir d'aldoses par application ou adaptation des méthodes décrites dans P. Collins, R. Ferrier, Monosaccharides, Their Chemistry and their roles in Natural Products, éditeur J. Wiley (1995), M. Bols, Carbohydrate Building Bloks, éditeur J. Wiley (1996).

Le stéréoisomère de l'amino-aldose de formule (XI) utilisé de façon préférentielle est la D-galactosamine.

Les stéréoisomères des composés de formule (XII) peuvent être ceux des cétoses à 6 atomes de carbone; ceux utlisés préférentiellement sont le D-psicose, le D-sorbose, le D-tagatose, le L-psicose, le L-fructose, le L-sorbose, le L-tagatose.

Les mélanges réactionnels obtenus par les divers procédés décrits précédemment sont traités suivant des méthodes classiques physiques (évaporation, extraction, distillation, chromatographie, cristallisation par exemple) ou chimiques (formation de sels par exemple).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-b-oxynaphtoate, chlorhydrate, sulfate, nitrate et phosphate.

Les exemples suivants illustrent plus particulièrement le procédé de préparation utilisé selon l'invention.

### EXEMPLE 1

Une solution de 1,0 g de D-sorbose et 3,5 g de formiate d'ammonium dans 4 cm³ d'eau est chauffée à reflux pendant 0,5 heure puis laissée refroidir à température ambiante. Le mélange est concentré sous pression réduite (2,7 kPa) à une température voisine de 40°C. Le résidu brun est repris successivement dans l'éther éthylique et le toluène et évaporé à sec. Le nouveau résidu est repris dans de l'éthanol et filtré. Le filtrat est évaporé pour donner une huile marron. L'opération est répétée à plusieurs reprises jusqu'à ce qu'il n'y ait plus de précipité. Le résidu ainsi obtenu est purifiée par chromatographie sur une colonne de silice (0,063-0,200 mm) en éluant avec un mélange éthanol/n-butanol/solution aqueuse d'ammoniaque à 28%/eau 8/2/2/1 en volumes. Les fractions contenant le produit attendu sont réunies et concentrées sous pression réduite (2,7 kPa) à une température voisine de 40°C. Le solide collant jaune obtenu est repris dans suffisamment d'éthanol/méthanol pour obtenir une solution, suivi de l'addition d'éther éthylique jusqu'au début de l'apparition d'un précipité qui est filtré. Le produit cristallise pour donner 0,15 g de 1-[5-(2R,3R,4-trihydroxy-butyl)-pyrazin-2-yl]-butane-1S,2R,3R,4-tetraol sous forme d'un solide beige qui fond à 90°C [Spectre de R.M.N. ¹H (400 MHz, (CD₃)₂SO d6, δ en ppm) : 2,85 et 2,93 (2dd, respectivement J = 13 et 9 Hz et J = 13 et 4 Hz, 2H : CH₂ 5α); de 3,25 à 3,55 (mt, 6H : CH 2γ - CH₂ 2δ - CH 5γ et CH₂ 5δ); 3,76 (mt, 1H : CH 2β); 3,91 (mt, 1H : CH 5β); de 4,35 à 4,65 (mf, 6H : OH en 2β - OH en 2γ - OH en 2δ - OH en 5β - OH en 5γ et OH en 5δ); 4,78 (t, J = 4,5 Hz, 1H : CH 2α); 5,39 (d, J = 4,5 Hz, 1H : OH en 2α); 8,43 (s, 1H : =CH en 6); 8,61 (s, 1H : =CH en 3). α_{D}²⁰= +71,3° ±1,3 (c=0,5% MeOH)]

### EXEMPLE 2

Une suspension contenant 1,0 g de D-galactosamine, chlorhydrate et 0,73 cm³ de diéthylamine est laissée sous agitation pendant 1 heure, puis filtrée. Le filtrat est évaporé et mis en solution dans 10 cm³ d'ammoniaque à 28% d'ammoniac et laissé sous agitation à température ambiante pendant trois semaines. Le mélange est alors concentré sous pression réduite (2,7 kPa) à une température voisine de 40°C pour donner une huile jaune qui est reprise dans du méthanol et filtrée. Le filtrat est évaporé pour donner une huile orange qui est purifiée par chromatographie sur une colonne de silice (0,04-0,063 mm) en éluant avec un mélange éthanol/n-butanol/ammoniaque à 28%/eau 8/2/2/1 en volumes. Les fractions contenant le produit attendu sont réunies et concentrées sous pression réduite (2,7 kPa) à une température voisine de 40°C pour donner un solide collant jaune orangé. Celui-ci est cristallisé dans du méthanol et le solide est filtré pour donner 0,12g de 1-[5-(1R,2R,3R,4-tétrahydroxy-butyl)-pyrazin-2-yl]-butane-1R,2R,3R,4-tétraol sous forme d'une poudre beige qui fond à 109°C [Spectre de R.M.N. ¹ H (400 MHz, (CD₃)₂SO d6, δ en ppm) : de 3,35 à 3,50 (mt, 4H : CH₂O 2δ et CH₂O 5δ); de 3,70 à 3,85 (mt, 4H : CH 2β - CH 2γ - CH 5β et CH 5γ); 4,24 (d, J = 8 Hz, 2H : OH en 2β et OH en 5β); 4,41 (d, J = 6,5 Hz, 2H : OH en 2γ et OH en 5γ); 4,50 (t large, J = 6 Hz, 2H : OH en 2δ et OH en 5δ); 4,64 (2 dd, J = 7 et 6 Hz, 2H : CH 2α et CH 5α); 5,48 (d, J = 6 Hz, 2H : OH en 2α et OH en 5α); 8,56 (s, 2H : =CH en 3 et =CH en 6)].

### EXEMPLE 3

Une solution de 1,0 g de D-tagatose et 3,5 g de formiate d'ammonium dans 4 cm³ d'eau est chauffée à reflux pendant 0,5 heure puis laissée refroidir à température ambiante. Le mélange est filtré et le résidu concentré sous pression réduite (2,7 kPa) à une température voisine de 40°C pour donner un résidu marron qui est reprise successivement dans l'éthanol et l'éther éthylique et évaporée à sec. Ce résidu est trituré dans l'éther éthylique et filtré. Le solide brun est solubilisé dans l'éthanol. A cette solution est ajouté de l'hydroxyde de sodium jusqu'à pH 12, et la solution est laissée sous agitation pendant 40 heures, on observe alors la formation d'un précipité. Le mélange réactionnel est filtré et le filtrat est concentré sous pression réduite (2,7 kPa) à une température voisine de 40°C pour donner un solide jaune qui est repris dans du méthanol/éther éthylique et filtré. Après évaporation du filtrat le résidu est dissous dans le méthanol et amené à pH 2 par addition d'éthanol chlorhydrique. Le précipité qui se forme est filtré et le filtrat est concentré. Le résidu est purifiée par chromatographie sur une colonne de silice (0,040-0,063 mm) en éluant avec un mélange éthanol/n-butanol/ammoniaque aqueuse/eau 8/2/1/1 en volumes. Les fractions contenant le produit attendu sont réunies et concentrées sous pression réduite (2,7 kPa) à une température voisine de 40°C. Le solide blanc ainsi obtenu est recristallisé dans le méthanol. On obtient90 mg de 1-[5-(2R,3R,4-trihydroxy-butyl)-pyrazin-2-yl]-butane-1R,2R,3R,4-tétraol sous forme d'un solide cristallin blanc qui fond à 146°C [Spectre de R.M.N. ¹H (400 MHz, (CD₃)₂SO d6, δ en ppm) : 2,86 (dd, J = 14 et 9 Hz, 1H : 1H du CH₂ 5α); 2,92 (dd, J = 14 et 3,5 Hz, 1H : l'autre H du CH₂ 5α); de 3,30 à 3,60 (mt, 5H : CH₂O 2δ - CH₂O 5δ et CH 5γ); de 3,70 à 3,85 (mt, 2H : CH 2γ et CH 2β); 3,90 (mt, 1H : CH 5β); 4,22 (d, J = 7 Hz, 1H : OH en 2β); 4,38 (d, J = 6,5 Hz, 1H : OH en 2γ); 4,43 (d, J = 7 Hz, 1H : OH en 5β); de 4,40 à 4,55 (mt, 2H : OH en 2δ et OH en 5δ); de 4,55 à 4,70 (mt, 2H : CH 2α et OH en 5γ); 5,44 (d, J = 6 Hz, 1H : OH en 2α); 8,43 (s, 1H : =CH en 6); 8,54 (s,1H : =CH en 3), α_{D}²⁰= -14,6° ± 1,13 (c=0,2% eau)].

### EXEMPLE 4

Une solution de 10,0 g de L-sorbose et 7,0 g de formiate d'ammonium dans 28 cm³ d'eau est chauffée à reflux pendant 2,5 heures puis laissée refroidir à température ambiante. Le mélange est concentré sous pression réduite (2,7 kPa) à une température voisine de 50°C. Le résidu pâteux marron est purifié par chromatographie sur une colonne de silice (0,020-0,045 mm) en éluant avec un mélange éthanol/n-butanol/solution aqueuse d'ammoniac/eau 8/2/2/1 en volumes. Les fractions contenant le produit attendu sont réunies et concentrées sous pression réduite (2,7 kPa) à une température voisine de 50°C. L'huile marron obtenue (9,1 g) est reprise dans un mélange de 100 cm³ d'éthanol et 10 cm³ d'eau. Le mélange est porté à reflux, traité avec 0,9 g de noir animal, puis filtré sur papier. Le filtrat est concentré sous pression réduite (2,7 kPa) à une température voisine de 50°C pour donner une huile marron (6,2 g). Cette dernière est reprise dans un mélange de 50 cm³ d'éthanol et 1,5 cm³ d'eau et recristallisée. Les cristaux obtenus sont filtrés, essorés puis lavés par le même mélange. Après séchage à poids constant, on obtient 0,86 g de 1-[5-(2S,3S,4-trihydroxy-butyl)-pyrazin-2-yl]-butane-1R,2S,3S,4-tétraol sous forme d'un solide cristallin beige fondant à 116°C. Spectre de R.M.N. ¹H (400 MHz, (CD₃)₂SO d6, δ en ppm) : 2,87 (AB limite, 2H : CH₂ 5α) ; de 3,30 à 3,60 (mt, 6H : CH 2γ - CH₂O 2δ-CH 5γ et CH₂O 5δ); 3,76 (mt, 1H : CH 2β) ; 3,90 (mt, 1H : CH 5β) ; 4,77 (d, J = 5,5 Hz, 1H : CH 2α) ; 8,43 (s large, 1H : =CH en 6) ; 8,61 (s large, 1H : =CH en 3). α_{D}²⁰= -62,4° +/-1,2 (c=0,5/ eau).

### EXEMPLE 5

Une solution de 5,0 g de L-gulose et 5,2 g de formiate d'ammonium dans 20 cm³ d'eau est chauffée à reflux pendant 2 heures puis laissée refroidir à température ambiante. Le mélange est concentré sous pression réduite (2,7 kPa) à une température voisine de 50°C. Le résidu pâteux noir est repris dans le méthanol, trituré, filtré et la fraction insoluble est lavée au méthanol. Le filtrat est concentré sous pression réduite (2,7 kPa) à une température voisine de 50°C pour donner 7,5 g d'une huile marron. Celle-ci est purifiée par chromatographie sur une colonne de silice (0,020-0,045 mm) en éluant avec un mélange éthanol/n-butanol/solution aqueuse d'ammoniac/eau 8/2/2/1 en volumes. Les fractions contenant le produit attendu sont réunies et concentrées sous pression réduite (2,7 kPa) à une température voisine de 50°C, reprises successivement dans l'éthanol et l'éther puis reconcentrées. L'huile obtenue (0,6 g) est reprise dans 5 cm³ d'eau puis lyophilisée. On obtient ainsi 0,47 g de 1-[6-(2S,3S,4-trihydroxy-butyl)-pyrazin-2-yl]-butane-1R,2S,3S,4-tétraol sous forme d'un lyophilisât brun. Spectre de R.M.N. ¹H (400 MHz, (CD₃)₂SO d6, à une température de 383 K, δ en ppm) : 2,94 et 3,03 (2 dd, respectivement J = 14 et 9 Hz et J = 14 et 4 Hz, 1H chacun : CH₂ 6α) ; de 3,40 à 3,70 (mt, 6H : CH 2γ - CH₂O 2δ - CH 6γ et CH₂O 6δ) ; 3,88 (t, J = 4 Hz, 1H : CH 2β) ; 4,01 (mt, 1H : CH 6β) ; 4,84 (d, J = 4 Hz, 1H : CH 2α) ; 8,42 (s, 1H : =CH en 5) ; 8,57 (s, 1H : =CH en 3). α_{D}²⁰= -65,9° +/-1,4 (c=0,5/eau).

### EXEMPLE 6

Une solution de 5,0 g de L-glucose et 8,8 g de formiate d'ammonium dans 14 cm³ d'eau est chauffée à reflux pendant 3 heures puis laissée refroidir à température ambiante. Le mélange est concentré sous pression réduite (2,7 kPa) à une température voisine de 65°C. Le résidu pâteux marron est repris dans le méthanol, trituré, filtré et la fraction insoluble est lavée au méthanol. Le filtrat est concentré sous pression réduite (2,7 kPa) à une température voisine de 50°C. Cette opération est répétée dans l'éthanol pour donner une huile marron (6,2 g). Celle-ci est purifiée par chromatographie sur une colonne de silice (0,020-0,045 mm) en éluant avec un mélange éthanol/n-butanol/solution aqueuse d'ammoniac 8/2/1 en volumes. Les fractions contenant le produit attendu sont réunies et concentrées sous pression réduite (2,7 kPa) à une température voisine de 60°C. L'huile obtenue (0,5 g) est reprise dans 14 cm³ d'éthanol, filtrée à chaud puis recristallisée. Les cristaux obtenus sont filtrés, lavés à l'éthanol puis essorés. Après séchage à poids constant à une température voisine de 40°C, on obtient 0,35 g de 1-[6-(2R,3S,4-trihydroxy-butyl)-pyrazin-2-yl]-butane-lS,2R,3S,4-tétraol sous forme d'un solide cristallin beige fondant à 114°C. (Rf=0,3; chromatographie sur couche mince de gel de silice; éluant mélange éthanol/ n-butanol/ solution aqueuse d'ammoniac/ eau 8:2:2:1 en volumes)].

### EXEMPLE 7

Une solution de 2,0 g de D-psicose et 3,2 g de formiate d'ammonium dans 3,4 cm³ d'eau est chauffée à reflux pendant 2 heures puis laissée refroidir à température ambiante. Le mélange est dilué avec 25 cm³ d'acétate d'éthyle et décanté. La phase aqueuse est lavée avec 25 cm³ d'acétate d'éthyle puis concentrée sous pression réduite (2,7 kPa) à une température voisine de 70°C. Le résidu huileux marron est repris dans 100 cm³ d'éthanol, trituré, filtré et la fraction insoluble est lavée à l'éthanol. Le filtrat est concentré sous pression réduite (2,7 kPa) à une température voisine de 45°C pour donner une pâte marron (1,6 g). Celle-ci est purifiée par chromatographie sur une colonne de silice (0,020-0,045 mm) en éluant avec un mélange éthanol/eau 199/1 en volumes, puis par chromatographie sur une colonne de silice (0,020-0,045 mm) en éluant avec un mélange acétate d'éthyle/acide acétique/eau 30/12/10 en volumes, et enfin par chromatographie sur une colonne de silice (0,020-0,045 mm) à une pression d'environ 1,5x10⁵ Pa en éluant avec un mélange éthanol/n-butanol/solution aqueuse d'ammoniac 8/2/1 en volumes. Les fractions contenant le produit attendu sont réunies et concentrées sous pression réduite (2,7 kPa) à une température voisine de 50°C. Le solide ambré obtenu (0,22 g) est repris dans un mélange de 5 cm³ d'éthanol et 0,25 cm³ d'eau, filtré à chaud puis recristallisé. Les cristaux obtenus sont filtrés, lavés à l'éthanol puis essorés. Après séchage à poids constant, on obtient 65,5 mg de 1-[5-(2S,3R,4-trihydroxy-butyl)-pyrazin-2-yl]-butane-1S,2S,3R,4-tétraol sous forme d'une poudre cristalline ocre fondant à 141°C. Spectre de R.M.N. ¹H (400 MHz, (CD₃)₂SO d6, δ en ppm) : 2,75 et 3,08 (2 dd, respectivement J = 14 et 10 Hz et J = 14 et 2,5 Hz, 1H chacun : CH₂ 5α) ; de 3,30 à 3,50 (mt, 4H : CH 2γ - CH 5γ - 1H du CH₂O 2δ et 1H du CH₂O 5δ) ; 3,60 (mt, 2H : l'autre H du CH₂O 2δ et l'autre H du CH₂O 5δ) ; 3,79 (mt, 2H : CH 2β et CH 5β) ; 4,36 et 4,45 (2 t, J = 5,5 Hz, 1H chacun : OH en 2δ et OH en 5δ) ; 4,58 - 4,64 - 4,71 et 4,78 (4 d, respectivement J = 4,5 Hz - J = 6,5 Hz - J = 5 Hz et J = 5,5 Hz, 4H : OH) ; 4,82 (t, J = 5,5 Hz, 1H : CH 2α) ; 5,53 (d, J = 5,5 Hz, 1H : OH en 2α) ; 8,41 (s large, 1H : =CH en 6) ; 8,60 (s large,1H : =CH en 3).

### EXEMPLE 8

Une solution de 5,0 g de D-galactose et 8,8 g de formiate d'ammonium dans 14 cm³ d'eau est chauffée à reflux pendant 45 minutes puis laissée refroidir à température ambiante. Le mélange est dilué avec 50 cm³ d'acétate d'éthyle et décanté. La phase aqueuse est lavée 2 fois avec 50 cm³ d'acétate d'éthyle puis concentrée sous pression réduite (2,7 kPa) à une température voisine de 65°C. Le résidu pâteux marron est repris dans 100 cm³ d'éthanol, trituré et le supernatant est concentré sous pression réduite (2,7 kPa) à une température voisine de 45°C (opération répétée une fois). Le solide marron résiduel est repris successivement dans le méthanol, l'éthanol, puis l'éther diéthylique et évaporé à sec sous pression réduite (2,7 kPa) à une température voisine de 45°C. Le résidu est purifié par chromatographie sur une colonne de silice (0,020-0,045 mm) à une pression d'environ 1,5x10⁵ Pa et en éluant avec un mélange éthanol/n-butanol/solution aqueuse d'ammoniac 8/2/1 en volumes. Les fractions contenant le produit attendu sont réunies et concentrées sous pression réduite (2,7 kPa) à une température voisine de 40°C. Le solide jaune ainsi obtenu (0,26 g) est repris dans un mélange de 3 cm³ d'éthanol et 0,25 cm³ d'eau, filtré à chaud puis recristallisé. Le solide obtenu est filtré, puis essoré. Après séchage sous pression réduite (2,7 kPa) à une température voisine de 25°C, on obtient 119 mg de 1-[6-(2R,3R,4-trihydroxy-butyl)-pyrazin-2-yl]-butane-1R,2R,3R,4-tétraol sous forme d'un solide pâteux ambré qui fond à 90-130°C (pâte). Spectre de R.M.N. ¹H (400 MHz, (CD₃)₂SO d6, δ en ppm) : 2,89 (AB limite, 2H : CH₂ 6α) ; de 3,30 à 3,55 (mt, 5H : CH₂O 2δ - CH₂O 6δ et CH 6γ) ; de 3,70 à 3,85 (mt, 2H : CH 2γ et CH 2β) ; 3,92 (mt, 1H : CH 6β) ; 4,64 (d, J = 8,5 Hz, 1H : CH 2α) ; 8,38 (s, 1H : =CH en 5) ; 8,45 (s,1H : =CH en 3).

Les composés de formule (I) présentent des propriétés pharmacologiques intéressantes. Ce sont des hypoglycémiants.

L'activité hypoglycémiante des composés de formule (I) a été déterminée sur la réponse hyperglycémique à l'administration de glucose par la voie orale chez la souris normoglycémique, selon le protocole suivant :

Des souris Swiss albinos pesant entre 22 et 26 g sont laissées à jeûn pendant 2 heures. A la fin de cette période, la glycémie est mesurée et, immédiatement après, une dose de glucose (2 g/kg) est administrée par voie orale. Trente minutes plus tard, la glycémie est mesurée encore une fois. Les souris qui répondent par une hyperglycémie supérieure à 170 mg/dl sont sélectionnées et utilisées pour détecter l'activité hypoglycémiante des composés selon l'invention.

Les souris ainsi choisies sont réparties en groupes d'au moins 10 animaux. Des groupes distincts reçoivent des doses de 3 à 50 mg/kg de produit dans un véhicule tel que l'eau ou un mélange de méthylcellulose/tween et eau une fois par jour par tubage gastrique . Le traitement dure 4 jours. Au 4 ^{ème} jour, après le dernier traitement, les animaux reçoivent une dose de glucose (2 g/kg) et la glycémie est mesurée 20 à 40 minutes plus tard. Le pourcentage d'inhibition de la réponse hyperglycémique à l'administration de glucose est calculée par rapport à la réponse mesurée dans le groupe traité par le véhicule.

Dans ce test, les composés selon l'invention présentent un pourcentage d'inhibition de la glycémie supérieur ou égal à 10%.

Les composés de formule générale (I) selon l'invention présentent une faible toxicité. Leur DL50 est supérieure à 2000 mg/kg par voie orale chez la souris.

En thérapeutique humaine, ces produits sont utiles dans la prévention et le traitement du diabète et notamment du diabète de type II (NID diabète), du diabète de l'obèse, du diabète de la cinquantaine, du diabète métapléthorique, du diabète du sujet âgé et du diabète léger. Ils peuvent être utilisés en complément de l'insulinothérapie dans le diabète insulino dépendant où ils permettent de diminuer progressivement la dose d'insuline, le diabète instable, le diabète insulinorésistant, en complément des sulfamides hypoglycémiants quand ceux-ci ne déterminent pas de baisse suffisante de la glycémie. Ces produits peuvent être utilisés également dans les complications du diabète telles que les hyperlipémies, les troubles du métabolisme lipidique, les dyslipémies, l'obésité. Ils sont aussi utiles dans la prévention et le traitement des lésions d'athérosclérose et leurs complications (coronopathies, infarctus du myocarde, cardiomyopathies, évolution de ces trois complications vers l'insuffisance ventriculaire gauche, artériopathies diverses, artérites des membres inférieurs avec claudication et évolution vers les ulcères et la gangrène, insuffisance vasculaire cérébrale et ses complications, impuissance sexuelle d'origine vasculaire), la rétinopathie diabétique et de toutes ses manifestations (augmentation de la perméabilité capillaire, dilatation et thrombose capillaire, microanévrismes, shunt artérioveineux, dilatation veineuse, hémorragies ponctiformes et maculaires, exudats, oedèmes maculaires, manifestations de la rétinopathie proliférante : néovaisseaux, cicatrices de rétinite proliférante, hémorragies du vitrée, décollement de la rétine), la cataracte diabétique, la neuropathie diabétique dans ses diverses formes (polyneuropathies périphériques et ses manifestations telles que paresthésies, hyperesthésies et douleurs, mononeuropathies, radiculopathies, neuropathies autonomes, amyotrophies diabétiques), les manifestations du pied diabétique (ulcères des extrémités inférieures et du pied), la néphropathie diabétique dans ses deux formes diffuse et nodulaire, l'athéromatose (élévation des HDL lipoproteines favorisant l'élimination du cholestérol à partir des plaques d'athérome, baisse des LDL lipoproteines, baisse du rapport LDL/HDL, inhibition de l'oxydation des LDL, diminution de l'adhésivité plaquettaire), des hyperlipémies et des dyslipémies (hypercholestérolémies, hypertriglycéridémies, normalisation du taux des acides gras, normalisation de l'uricémie, normalisation des apoprotéines A et B), de la cataracte, de l'hypertension artérielle et ses conséquences.

Les médicaments selon l'invention sont constitués par un composé selon l'invention ou une combinaison de ces produits, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 150 mg et 600 mg par jour par voie orale pour un adulte avec des doses unitaires allant de 50 mg à 200 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Produit actif 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 50 mg de produit actif ayant la composition suivante :
- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau 4 ml

L'invention concerne également l'utilisation des composés de formule générale (I) pour la préparation de compositions pharmaceutiques utiles pour le traitement ou la prévention du diabète et les complications du diabète.

## Revendications

1. Médicaments contenant en tant que principe actif au moins un composé de formule générale : 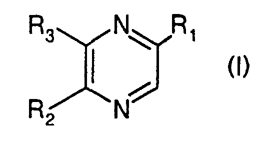 dans laquelle
R₁ représente les formes stéréoisomères de la chaîne
-(CHOH)₃-CH₂OH (II)
et
soit R₂ représente un atome d'hydrogène et R₃ représente les formes stéréoisomères de la chaîne
-CH₂-(CHOH)₂-CH₂OH (III)
soit R₂ représente les formes stéréoisomères des chaînes
-(CHOH)₃-CH₂OH (II)
ou
-CH₂-(CHOH)₂-CH₂OH (III)
et R₃ représente un atome d'hydrogène
à l'exception de
- la fructosazine de formule 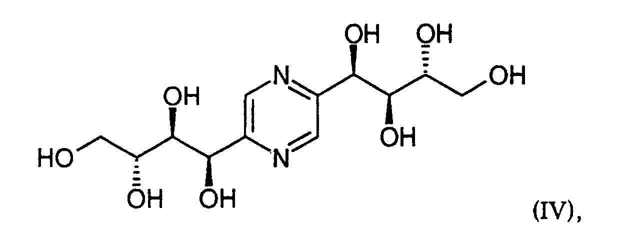
- la déoxyfructosazine de formule 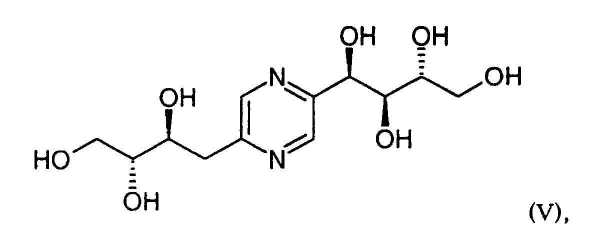
- et du composé de formule 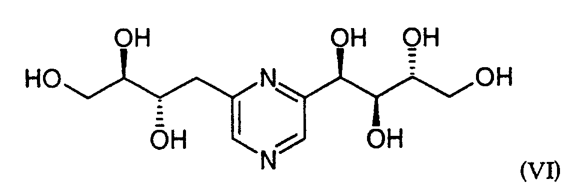
ou un sel d'un tel composé avec un acide organique ou minéral.

2. Médicaments selon la revendication 1 pour lequel le composé de formule (I) est choisi parmi :
1-[5-(1R,2R,3R,4-tétrahydroxy-butyl)-pyrazin-2-yl]-butane-1R,2R,3R,4-tétraol
1-[5-(1R,2R,3S,4-tétrahydroxy-butyl)-pyrazin-2-yl]-butane-1R,2R,3S,4-tétraol
1-[5-(1R,2S,3S,4-tétrahydroxy-butyl)-pyrazin-2-yl]-butane-1R,2S,3S,4-tétraol
1-[5-(1S,2R,3R,4-tétrahydroxy-butyl)-pyrazin-2-yl]-butane-1S,2R,3R,4-tétraol
1-[5-(1S,2R,3S,4-tétrahydroxy-butyl)-pyrazin-2-yl]-butane-1S,2R,3S,4-tétraol
1-[5-(1S,2S,3R,4-tétrahydroxy-butyl)-pyrazin-2-yl]-butane-1S,2S,3R,4-tétraol
1-[5-(1S,2S,3S,4-tétrahydroxy-butyl)-pyrazin-2-yl]-butane-1S,2S,3S,4-tétraol
1-[5-(2R,3R,4-trihydroxy-butyl)-pyrazin-2-yl]-butane-1R,2R,3R,4-tétraol
1-[5-(2R,3S,4-trihydroxy-butyl)-pyrazin-2-yl]-butane-1R,2R,3S,4-tétraol
1-[5-(2S,3S,4-trihydroxy-butyl)-pyrazin-2-yl]-butane-1R,2S,3S,4-tétraol
1-[5-(2R,3R,4-trihydroxy-butyl)-pyrazin-2-yl]-butane- 1S,2R,3R,4-tétraol
1-[5-(2R,3S,4-trihydroxy-butyl)-pyrazin-2-yl]-butane-1S,2R,3S,4-tétraol
1-[5-(2S,3R,4-trihydroxy-butyl)-pyrazin-2-yl]-butane-1S,2S,3R,4-tétraol
1-[5-(2S,3S,4-trihydroxy-butyl)-pyrazin-2-yl]-butane-1S,2S,3S,4-tétraol
1-[6-(2R,3R,4-trihydroxy-butyl)-pyrazin-2-yl]-butane-1R,2R,3R,4-tétraol
1-[6-(2R,3S,4-trihydroxy-butyl)-pyrazin-2-yl]-butane-1R,2R,3S,4-tétraol
1-[6-(2R,3R,4-trihydroxy-butyl)-pyrazin-2-yl]-butane-1S,2R,3R,4-tétraol
1-[6-(2R,3S,4-trihydroxy-butyl)-pyrazin-2-yl]-butane-1S,2R,3S,4-tétraol
1-[6-(2S,3R,4-trihydroxy-butyl)-pyrazin-2-yl]-butane-1S,2S,3R,4-tétraol
1-[6-(2S,3S,4-trihydroxy-butyl)-pyrazin-2-yl]-butane-1S,2S,3S,4-tétraol
ou un sel d'un tel composé avec un acide minéral ou organique.

3. Composés de formule : 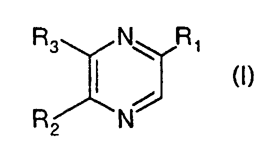 dans laquelle
R₁ représente les formes stéréoisomères de la chaîne
-(CHOH)₃-CH₂OH (II)
et
soit R₂ représente un atome d'hydrogène et R₃ représente les formes stéréoisomères de la chaîne
-CH₂-(CHOH)₂-CH₂OH (III)
soit R₂ représente les formes stéréoisomères des chaînes
-(CHOH)₃-CH₂OH (II)
ou
-CH₂-(CHOH)₂-CH₂OH (III)
et R₃ représente un atome d'hydrogène
ou leurs sels avec un acide minéral ou organique,
à l'exception des composés de formule :

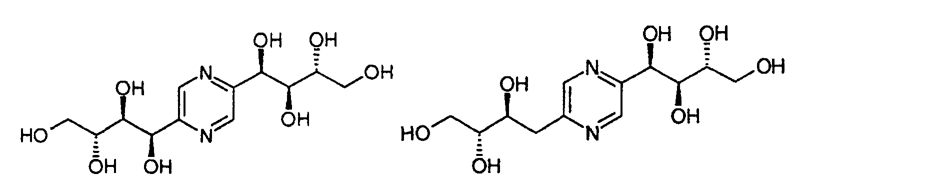

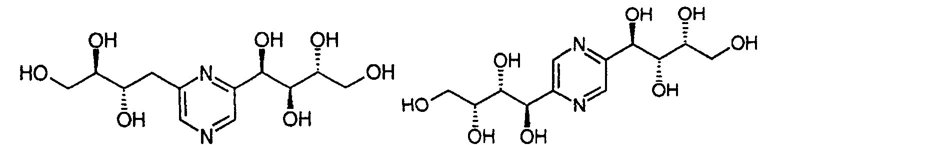

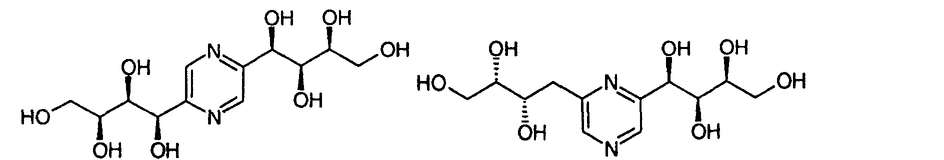

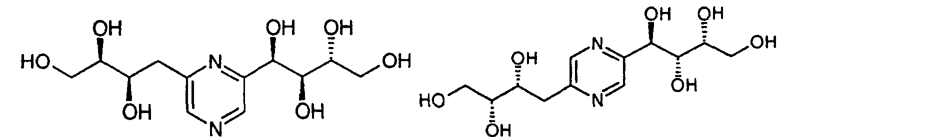

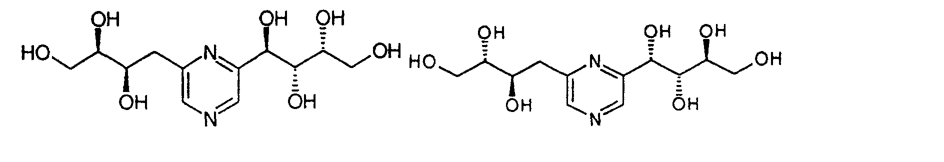

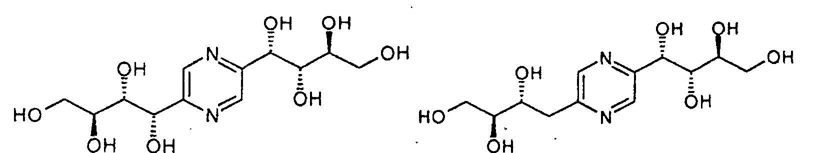

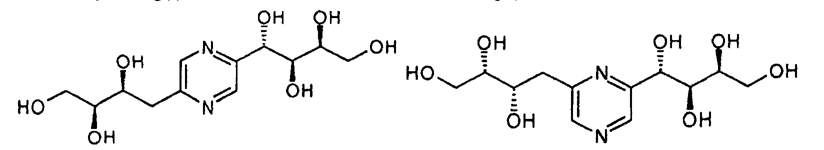

4. Composés de formule (I) selon la revendication 3 suivants :
1-[5-(1R,2R,3S,4-tétrahydroxy-butyl)-pyrazin-2-yl]-butane-1R,2R,3S,4-tétraol
1-[5-(1S,2R,3R,4-tétrahydroxy-butyl)-pyrazin-2-yl]-butane-1S,2R,3R,4-tétraol
1-[5-(1S,2S,3R,4-tétrahydroxy-butyl)-pyrazin-2-yl]-butane-1S,2S,3R,4-tétraol
1-[5-(1S,2S,3S,4-tétrahydroxy-butyl)-pyrazin-2-yl]-butane-1S,2S,3S,4-tétraol
1-[5-(2R,3S,4-trihydroxy-butyl)-pyrazin-2-yl]-butane-1R,2R,3S,4-tétraol
1-[5-(2S,3S,4-trihydroxy-butyl)-pyrazin-2-yl]-butane-1R,2S,3S,4-tétraol
1-[5-(2R,3R,4-trihydroxy-butyl)-pyrazin-2-yl]-butane-1S,2R,3R,4-tétraol
1-[5-(2S,3R,4-trihydroxy-butyl)-pyrazin-2-yl]-butane-1S,2S,3R,4-tétraol
1-[6-(2R,3S,4-trihydroxy-butyl)-pyrazin-2-yl]-butane-1R,2R,3S,4-tétraol
1-[6-(2R,3R,4-trihydroxy-butyl)-pyrazin-2-yl] -butane-1S,2R,3R,4-tétraol
1-[6-(2S,3R,4-trihydroxy-butyl)-pyrazin-2-yl]-butane-1S,2S,3R,4-tétraol
ou un sel d'un tel composé avec un acide minéral ou organique.

5. Composés de formule (I) selon la revendication 3 suivants :
1-[5-(2S,3S,4-trihydroxy-butyl)-pyrazin-2-yl]-butane-1R,2S,3S,4-tétraol
1-[5-(2R,3R,4-trihydroxy-butyl)-pyrazin-2-yl]-butane-1S,2R,3R,4-tétraol
1-[5-(2S,3R,4-trihydroxy-butyl)-pyrazin-2-yl]-butane-1S,2S,3R,4-tétraol et leurs sels avec un acide minéral ou organique.

6. Procédé de préparation des composés de formule (I) selon la revendication 3 pour lesquels R₁ représente les formes stéréoisomères de la chaîne -(CHOH)₃-CH₂OH (II), R₂ représente un atome d'hydrogène et R₃ représente les formes stéréoisomères de la chaîne -CH₂-(CHOH)₂-CH₂OH (III) **caractérisé en ce que** l'on fait réagir du formiate d'ammonium sur un aldose, ou un mélange de 2 aldoses, de série dextrogyre ou lévogyre de formule générale :
CHO-CHOH-R₁ (X)
dans laquelle R₁ a la même signification que dans la revendication 3, isole le produit et le transforme éventuellement en sel.

7. Procédé selon la revendication 6 pour lequel le dérivé de formule (X) est le D-gulose, le D-galactose, le D-allose, le D-altrose, le D-idose, le D-talose, le L-glucose, le L-mannose, le L-galactose, le L-allose, le L-altrose, le L-idose, le L-talose ou le L-gulose.

8. Procédé de préparation des composés de formule (I) selon la revendication 3 pour lesquels R₁ représente les formes stéréoisomères de la chaîne -(CHOH)₃-CH₂OH (II), R₂ représente les formes stéréoisomères des chaînes -(CHOH)₃-CH₂OH (II) et R₃ représente un atome d'hydrogène **caractérisé en ce que** l'on traite un amino-aldose, ou un mélange de 2 amino-aldoses, de formule générale CHO-CH(NH₂)-R₁ (XI) dans laquelle R₁ a la même signification que dans la revendication 3 en milieu basique, isole le produit et le transforme éventuellement en sel avec un acide minéral ou organique.

9. Procédé selon la revendication 8 pour lequel l'amino-aldose de formule (XI) utilisé est la D-galactosamine.

10. Procédé de préparation des composés de formule (I) selon la revendication 3 pour lesquels R₁ représente les formes stéréoisomères de la chaîne -(CHOH)₃-CH₂OH (II), R₂ représente les formes stéréoisomères des chaînes -CH₂-(CHOH)₂-CH₂OH (III)et R₃ représente un atome d'hydrogène **caractérisé en ce que** l'on traite un amino-aldose, ou un mélange de 2 amino-aldoses, de formule générale CHO-CH(NH₂)-R₁ (XI) dans laquelle R₁ a la même signification que dans la revendication 3, en milieu acide, isole le produit et le transforme éventuellement en sel avec un acide minéral ou organique.

11. Procédé selon la revendication 10 pour lequel le dérivé de formule (XI) est la D-galactosamine.

12. Procédé de préparation des composés de formule (I) selon la revendication 3 pour lesquels R₁ représente les formes stéréoisomères de la chaîne -(CHOH)₃-CH₂OH (II), R₂ représente les formes stéréoisomères des chaînes -CH₂-(CHOH)₂-CH₂OH (III) et R₃ représente un atome d'hydrogène **caractérisé en ce que** l'on fait réagir un cétose, ou un mélange de 2 cétoses de formule générale HOCH₂-CO-R₁ (XII) dans laquelle R₁ a la même signification que dans la revendication 3 et du formiate d'ammonium, isole le produit et le transforme éventuellement en sel avec un acide minéral ou organique.

13. Procédé selon la revendication 12 pour lequel le dérivé de formule (XII) est le D-psicose, le D-sorbose, le D-tagatose, le L-psicose, le L-fructose, le L-sorbose ou le L-tagatose.

14. Utilisation des composés de formule générale (I) 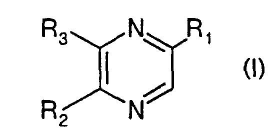 dans laquelle
R₁ représente les formes stéréoisomères de la chaîne
-(CHOH)₃-CH₂OH (II)
et
soit R₂ représente un atome d'hydrogène et R₃ représente les formes stéréoisomères de la chaîne
-CH₂-(CHOH)₂-CH₂OH (III)
soit R₂ représente les formes stéréoisomères des chaînes
-(CHOH)₃-CH₂OH (II)
ou
-CH₂-(CHOH)₂-CH₂OH (III)
et R₃ représente un atome d'hydrogène
ou leurs sels avec un acide minéral ou organique,
à l'exception de
- la fructosazine de formule 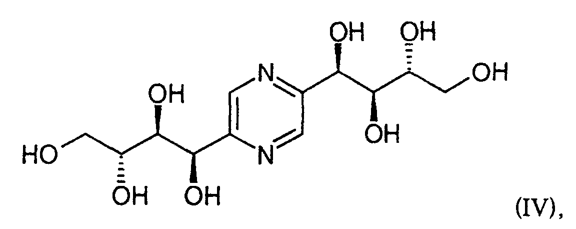
- la déoxyfructosazine de formule 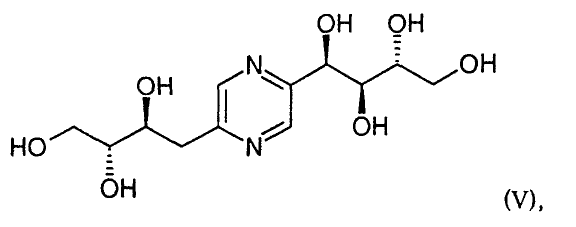
- et du composé de formule 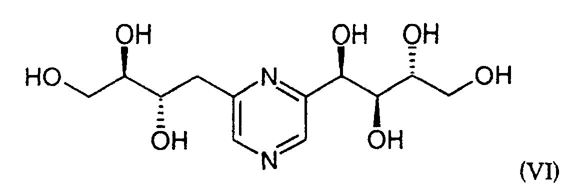 pour la préparation de compositions pharmaceutiques utiles pour le traitement ou la prévention du diabète et les complications du diabète.

## Patentansprüche

1. Arzneimittel, enthaltend als Wirkstoff mindestens eine Verbindung der allgemeinen Formel (I) 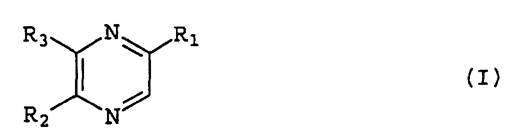 in der
R₁ die stereoisomeren Formen der Kette
-(CHOH)₃-CH₂OH (II)
darstellt und
R₂ entweder ein Wasserstoffatom bedeutet und R₃ die stereoisomeren Formen der Kette
-CH₂-(CHOH)₂-CH₂OH (III)
darstellt oder
R₂ die stereoisomeren Formen der Ketten
-(CHOH)₃-CH₂OH (II)
oder
-CH₂-(CHOH)₂-CH₂OH (III)
darstellt und
R₃ ein Wasserstoffatom bedeutet,
mit Ausnahme von
- Fructosazin der Formel (IV) 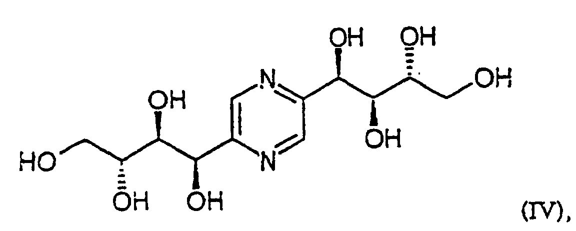
- Deoxyfructosazin der Formel (V) 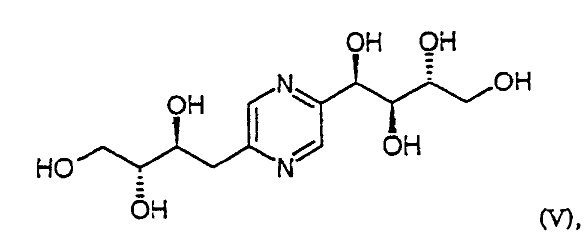
- und der Verbindung der Formel (VI) 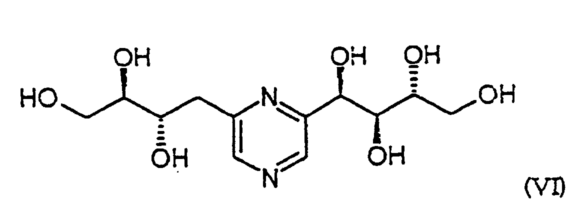
oder ein Salz einer derartigen Verbindung mit einer Mineralsäure oder organischen Säure.

2. Arzneimittel nach Anspruch 1, bei dem die Verbindung der Formel (I) ausgewählt wird unter:
1-[5-(1R,2R,3R,4-Tetrahydroxy-butyl)-pyrazin-2-yl]-butan-1R,2R,3R,4-tetraol,
1-[5-(1R,2R,3S,4-Tetrahydroxy-butyl)-pyrazin-2-yl]-butan-1R,2R,3S,4-tetraol,
1-[5-(1R,2S,3S,4-Tetrahydroxy-butyl) -pyrazin-2-yl]-butan-1R,2S,3S,4-tetraol,
1-[5-(1S,2R,3R,4-Tetrahydroxy-butyl)-pyrazin-2-yl]-butan-1S,2R,3R,4-tetraol,
1-[5-(1S,2R,3S,4-Tetrahydroxy-butyl)-pyrazin-2-yl)-butan-1S,2R,3S,4-tetraol,
1-[5-(1S,2S,3R,4-Tetrahydroxy-butyl)-pyrazin-2-yl]-butan-1S,2S,3R,4-tetraol,
1-[5-(1S,2S,3S,4-Tetrahydroxy-butyl)-pyrazin-2-yl]-butan-1S,2S,3S,4-tetraol,
1-[5-(2R,3R,4-Trihydroxy-butyl)-pyrazin-2-yl]-butan-1R,2R,3R,4-tetraol,
1-[5-(2R,3S,4-Trihydroxy-butyl)-pyrazin-2-yl]-butan-1R,2R,3S,4-tetraol,
1-[5-(2S,3S,4-Trihydroxy-butyl)-pyrazin-2-yl]-butan-1R,2S,3S,4-tetraol,
1-[5-(2R,3R,4-Trihydroxy-butyl)-pyrazin-2-yl]-butan-1S,2R,3R,4-tetraol,
1-[5-(2R,3S,4-Trihydroxy-butyl)-pyrazin-2-yl]-butan-1S,2R,3S,4-tetraol,
1-[5-(2S,3R,4-Trihydroxy-butyl)-pyrazin-2-yl]-butan-1S,2S,3R,4-tetraol,
1-[5-(2S,3S,4-Trihydroxy-butyl)-pyrazin-2-yl]-butan-1S,2S,3S,4-tetraol,
1-[6-(2R,3R,4-Trihydroxy-butyl)-pyrazin-2-yl]-butan-1R,2R,3R,4-tetraol,
1-[6-(2R,3S,4-Trihydroxy-butyl)-pyrazin-2-yl]-butan-1R,2R,3S,4-tetraol,
1-[6-(2R,3R,4-Trihydroxy-butyl)-pyrazin-2-yl]-butan-1S,2R,3R,4-tetraol,
1-[6-(2R,3S,4-Trihydroxy-butyl)-pyrazin-2-yl]-butan-1S,2R,3S,4-tetraol,
1-[6-(2S,3R,4-Trihydroxy-butyl)-pyrazin-2-yl]-butan-1S,2S,3R,4-tetraol,
1-[6-(2S,3S,4-Trihydroxy-butyl)-pyrazin-2-yl]-butan-1S,2S,3S,4-tetraol,
oder einem Salz einer derartigen Verbindung mit einer Mineralsäure oder organischen Säure.

3. Verbindungen der Formel (I) 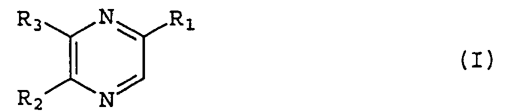 in der
R₁ die stereoisomeren Formen der Kette
-(CHOH)₃-CH₂OH (II)
darstellt und
R₂ entweder ein Wasserstoffatom bedeutet und R₃ die stereoisomeren Formen der Kette
-CH₂-(CHOH)₂-CH₂OH (III)
darstellt oder
R₂ die stereoisomeren Formen der Ketten
-(CHOH)₃-CH₂OH (II)
oder
-CH₂-(CHOH)₂-CH₂OH (III)
darstellt und
R₃ ein Wasserstoffatom bedeutet,
oder ihre Salze mit einer Mineralsäure oder organischen Säure, mit Ausnahme der Verbindungen der Formeln:

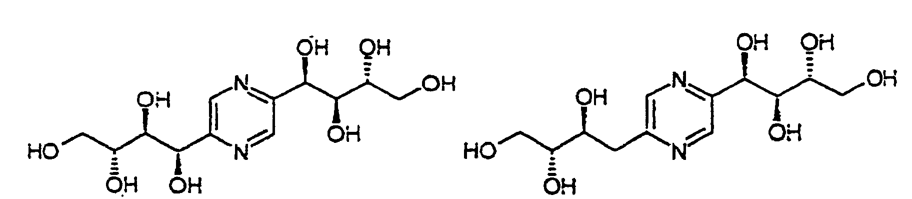

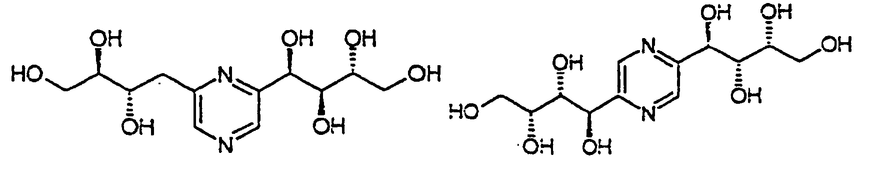

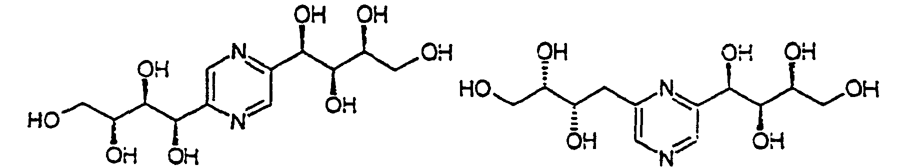

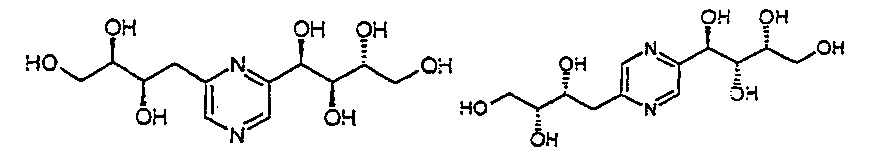

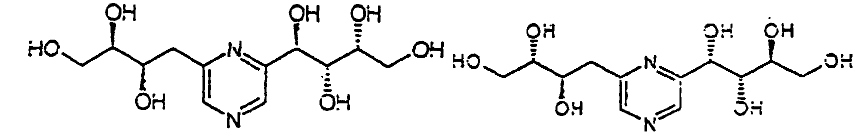

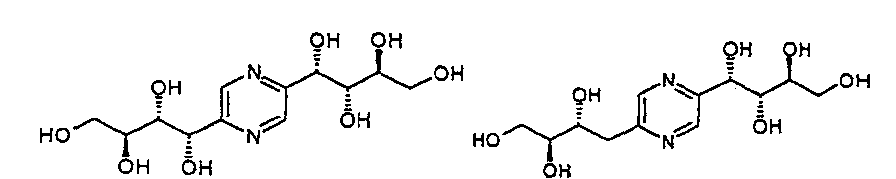

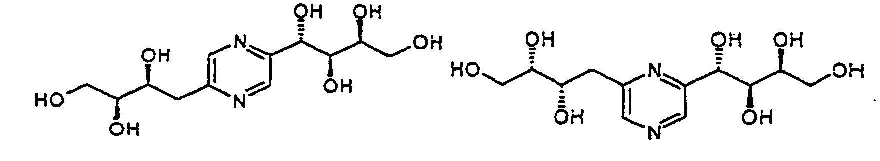

4. Verbindungen der Formel (I) nach Anspruch 3, die folgen:
1-[5-(1R,2R,3S,4-Tetrahydroxy-butyl)-pyrazin-2-yl]-butan-1R,2R,3S,4-tetraol,
1-[5-(1S,2R,3R,4-Tetrahydroxy-butyl)-pyrazin-2-yl]-butan-1S,2R,3R,4-tetraol,
1-[5-(1S,2S,3R,4-Tetrahydroxy-butyl)-pyrazin-2-yl]-butan-1S,2S,3R,4-tetraol,
1-[5-(1S,2S,3S,4-Tetrahydroxy-butyl)-pyrazin-2-yl]-butan-1S,2S,3S,4-tetraol,
1-[5-(2R,3S,4-Trihydroxy-butyl)-pyrazin-2-yl]-butan-1R,2R,3S,4-tetraol,
1-[5-(2S,3S,4-Trihydroxy-butyl)-pyrazin-2-yl]-butan-1R,2S,3S,4-tetraol,
1-[5-(2R,3R,4-Trihydroxy-butyl)-pyrazin-2-yl]-butan-1S,2R,3R,4-tetraol,
1-[5-(2S,3R,4-Trihydroxy-butyl)-pyrazin-2-yl]-butan-1S,2S,3R,4-tetraol,
1-[6-(2R,3S,4-Trihydroxy-butyl)-pyrazin-2-yl]-butan-1R,2R,3S,4-tetraol,
1-[6-(2R,3R,4-Trihydroxy-butyl) -pyrazin-2-yl]-butan-1S,2R,3R,4-tetraol,
1-[6-(2S,3R,4-Trihydroxy-butyl) -pyrazin-2-yl] -butan-1S,2S,3R,4-tetraol,
oder ein Salz einer derartigen Verbindung mit einer Mineralsäure oder organischen Säure.

5. Verbindungen der Formel (I) nach Anspruch 3, die folgen:
1-[5-(2S,3S,4-Trihydroxy-butyl)-pyrazin-2-yl]-butan-1R,2S,3S,4-tetraol,
1-[5-(2R,3R,4-Trihydroxy-butyl) -pyrazin-2-yl]-butan-1S,2R,3R,4-tetraol,
1-[5-(2S,3R,4-Trihydroxy-butyl)-pyrazin-2-yl]-butan-1S,2S,3R,4-tetraol,
und ihre Salze mit einer Mineralsäure oder organischen Säure.

6. Verfahren zur Herstellung der Verbindungen der Formel (I) nach Anspruch 3, worin R₁ die stereoisomeren Formen der Kette -(CHOH)₃-CH₂OH (II) darstellt, R₂ ein Wasserstoffatom bedeutet und R₃ die stereoisomeren Formen der Kette -CH₂-(CHOH)₂-CH₂OH (III) darstellt, **dadurch gekennzeichnet, daß** man Ammoniumformiat mit einer Aldose oder mit einer Mischung von zwei Aldosen der rechtsdrehenden Reihe oder der linksdrehenden Reihe der allgemeinen Formel (X)
CHO-CHOH-R₁ (X)
zur Reaktion bringt, in der R₁ die gleiche Bedeutung wie in Anspruch 3 besitzt, das Produkt isoliert und gegebenenfalls in Salz überführt.

7. Verfahren nach Anspruch 6, worin das Derivat der Formel (X) D-Gulose, D-Galactose, D-Allose, D-Altrose, D-Idose, D-Talose, L-Glucose, L-Mannose, L-Galactose, L-Allose, L-Altrose, L-Idose, L-Talose oder L-Gulose ist.

8. Verfahren zur Herstellung der Verbindungen der Formel (I) nach Anspruch 3, worin R₁ die stereoisomeren Formen der Kette -(CHOH)₃-CH₂OH (II) darstellt, R₂ die stereoisomeren Formen der Ketten -(CHOH)₃-CH₂OH (II) bedeutet und R₃ ein Wasserstoffatom darstellt, **dadurch gekennzeichnet, daß** man eine Aminoaldose oder eine Mischung von zwei Aminoaldosen der allgemeinen Formel CHO-CH(NH₂)-R₁ (XI), worin R₁ die gleiche Bedeutung wie in Anspruch 3 besitzt, im basischen Medium behandelt, das Produkt isoliert und gegebenenfalls in ein Salz mit einer Mineralsäure oder organischen Säure überführt.

9. Verfahren nach Anspruch 8, bei dem die verwendete Aminoaldose der Formel (XI) das D-Galactosamin ist.

10. Verfahren zur Herstellung der Verbindungen der Formel (I) nach Anspruch 3, worin R₁ die stereoisomeren Formen der Kette -(CHOH)₃-CH₂OH (II) darstellt, R₂ die stereoisomeren Formen der Ketten -CH₂-(CHOH)₂-CH₂OH (III) bedeutet und R₃ ein Wasserstoffatom darstellt, **dadurch gekennzeichnet, daß** man eine Aminoaldose oder eine Mischung von zwei Aminoaldosen der allgemeinen Formel CHO-CH(NH₂)-R₁ (XI), worin R₁ die gleiche Bedeutung wie in Anspruch 3 besitzt, im sauren Medium behandelt, das Produkt isoliert und gegebenenfalls in ein Salz mit einer Mineralsäure oder organischen Säure überführt.

11. Verfahren nach Anspruch 10, bei dem das Derivat der Formel (XI) das D-Galactosamin ist.

12. Verfahren zur Herstellung der Verbindungen der Formel (I) nach Anspruch 3, worin R₁ die stereoisomeren Formen der Kette -(CHOH)₃-CH₂OH (II) darstellt, R₂ die stereoisomeren Formen der Ketten -CH₂-(CHOH)₂-CH₂OH (III) bedeutet und R₃ ein Wasserstoffatom darstellt, **dadurch gekennzeichnet, daß** man eine Ketose oder eine Mischung von zwei Ketosen der allgemeinen Formel HOCH₂-CO-R₁ (XII), worin R₁ die gleiche Bedeutung wie in Anspruch 3 besitzt, und Ammoniumformiat zur Reaktion bringt, das Produkt isoliert und gegebenenfalls in ein Salz mit einer Mineralsäure oder organischen Säure überführt.

13. Verfahren nach Anspruch 12, **dadurch gekennzeichnet, daß** das Derivat der Formel (XII) D-Psicose, D-Sorbose, D-Tagatose, L-Psicose, L-Fructose, L-Sorbose oder L-Tagatose ist.

14. Verwendung von Verbindungen der allgemeinen Formel (I) 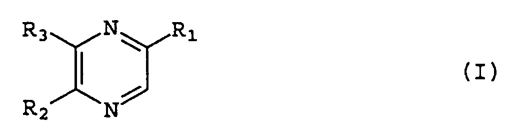 in der
R₁ die stereoisomeren Formen der Kette
-(CHOH)₃-CH₂OH (II)
darstellt und
R₂ entweder ein Wasserstoffatom bedeutet und R₃ die stereoisomeren Formen der Kette
-CH₂-(CHOH)₂-CH₂OH (III)
darstellt oder
R₂ die stereoisomeren Formen der Ketten
-(CHOH)₃-CH₂OH (II)
oder
-CH₂-(CHOH)₂-CH₂OH (III)
darstellt und
R₃ ein Wasserstoffatom bedeutet,
oder ihren Salzen mit einer Mineralsäure oder organischen Säure, mit Ausnahme von
- Fructosazin der Formel (IV) 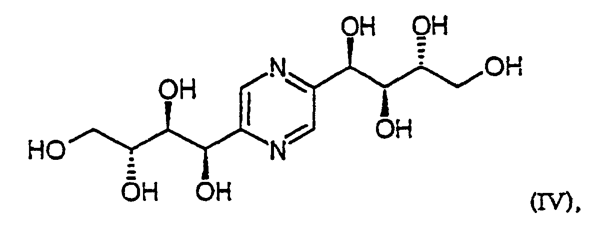
- Deoxyfructosazin der Formel (V) 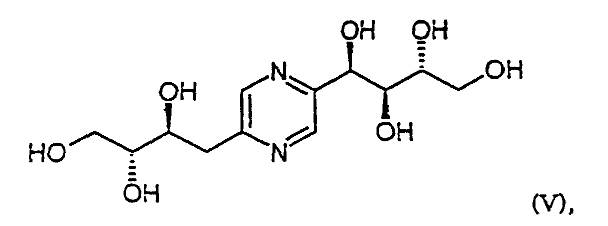
- und der Verbindung der Formel (VI) 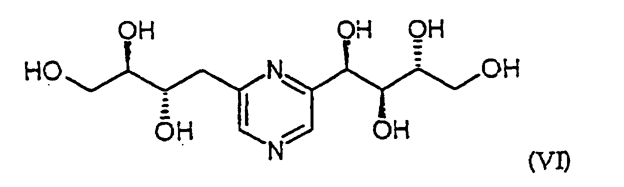
für die Herstellung von pharmazeutischen Zusammensetzungen zur Behandlung oder Vorbeugung von Diabetes und von Komplikationen von Diabetes.

## Claims

1. Medicaments comprising, as active principle, at least one compound of general formula: 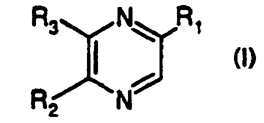 in which
R₁ represents the stereoisomeric forms of the chain
-(CHOH)₃-CH₂OH (II)
and
either R₂ represents a hydrogen atom and R₃ represents the stereoisomeric forms of the chain
-CH₂-(CHOH)₂-CH₂OH (III)
or R₂ represents the stereoisomeric forms of the chains
-(CHOH)₃-CH₂OH (II)
or
-CH₂-(CHOH)₂-CH₂OH (III)
and R₃ represents a hydrogen atom
with the exception of
- fructosazine of formula 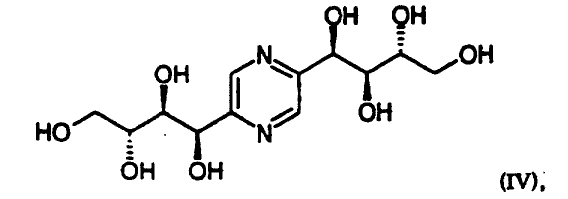
- deoxyfructosazine of formula 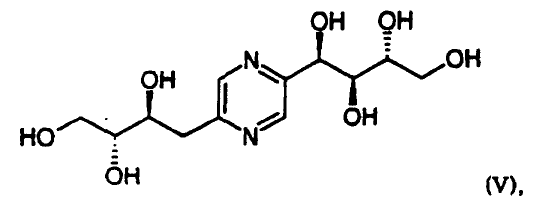
- and the compound of formula 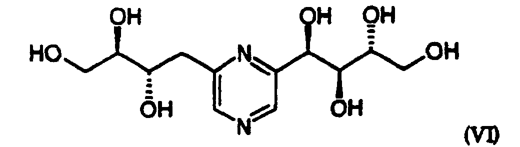
or a salt of such a compound with an organic or inorganic acid.

2. Medicaments according to claim 1 in which the compound of formula (I) is chosen from:
1-[5-(1R,2R,3R,4-tetrahydroxybutyl)pyrazin-2-yl]butane-1R,2R,3R,4-tetraol
1-[5-(1R,2R,3S,4-tetrahydroxybutyl)pyrazin-2-yl]butane-1R,2R,3S,4-tetraol
1-[5-(1R,2S,3S,4-tetrahydroxybutyl)pyrazin-2-yl]butane-1R,2S,3S,4-tetraol
1-[5-(1S,2R,3R,4-tetrahydroxybutyl)pyrazin-2-yl]butane-1S,2R,3R,4-tetraol
1-[5-(1S,2R,3S,4-tetrahydroxybutyl)pyrazin-2-yl]butane-1S,2R,3S,4-tetraol
1-[5-(1S,2S,3R,4-tetrahydroxybutyl)pyrazin-2-yl]butane-1S,2S,3R,4-tetraol
1-[5-(1S,2S,3S,4-tetrahydroxybutyl)pyrazin-2-yl]butane-1S,2S,3S,4-tetraol
1-[5-(2R,3R,4-trihydroxybutyl)pyrazin-2-yl]butane-1R,2R,3R,4-tetraol
1-[5-(2R,3S,4-trihydroxybutyl)pyrazin-2-yl]butane-1R,2R,3S,4-tetraol
1-[5-(2S,3S,4-trihydroxybutyl)pyrazin-2-yl]butane-1R,2S,3S,4-tetraol
1-[5-(2R,3R,4-trihydroxybutyl)pyrazin-2-yl]butane-1S,2R,3R,4-tetraol
1-[5-(2R,3S,4-trihydroxybutyl)pyrazin-2-yl]butane-1S,2R,3S,4-tetraol
1-[5-(2S,3R,4-trihydroxybutyl)pyrazin-2-yl]butane-1S,2S,3R,4-tetraol
1-[5-(2S,3S,4-trihydroxybutyl)pyrazin-2-yl]butane-1S,2S,3S,4-tetraol
1-[6-(2R,3R,4-trihydroxybutyl)pyrazin-2-yl]butane-1R,2R,3R,4-tetraol
1-[6-(2R,3S,4-trihydroxybutyl)pyrazin-2-yl]butane-1R,2R,3S,4-tetraol
1-[6-(2R,3R,4-trihydroxybutyl)pyrazin-2-yl]butane-1S,2R,3R,4-tetraol
1-[6-(2R,3S,4-trihydroxybutyl)pyrazin-2-yl]butane-1S,2R,3S,4-tetraol
1-[6-(2S,3R,4-trihydroxybutyl)pyrazin-2-yl]butane-1S,2S,3R,4-tetraol
1-[6-(2S,3S,4-trihydroxybutyl)pyrazin-2-yl]butane-1S,2S,3S,4-tetraol
or a salt of such a compound with an inorganic or organic acid.

3. Compounds of formula: 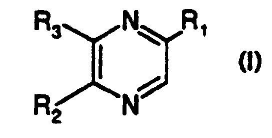 in which
R₁ represents the stereoisomeric forms of the chain
-(CHOH)₃-CH₂OH (II)
and
either R₂ represents a hydrogen atom and R₃ represents the stereoisomeric forms of the chain
-CH₂-(CHOH)₂-CH₂OH (III)
or R₂ represents the stereoisomeric forms of the chains
-(CHOH)₃-CH₂OH (II)
or
-CH₂-(CHOH)₂-CH₂OH (III)
and R₃ represents a hydrogen atom,
or their salts with an inorganic or organic acid, with the exception of the compounds of formula:

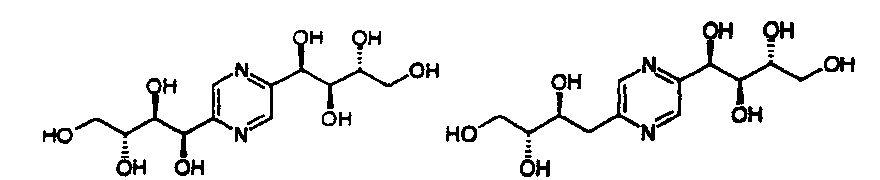

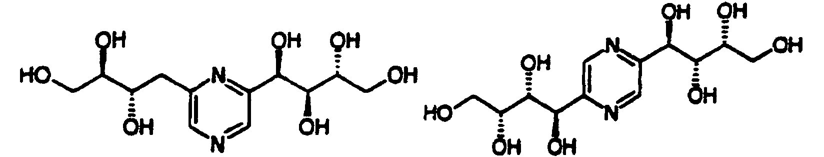

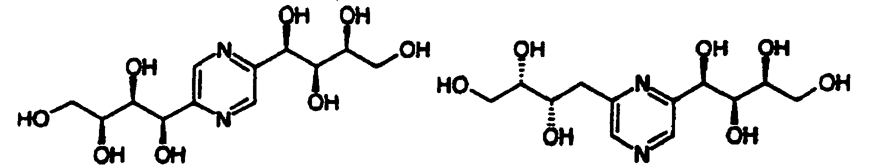

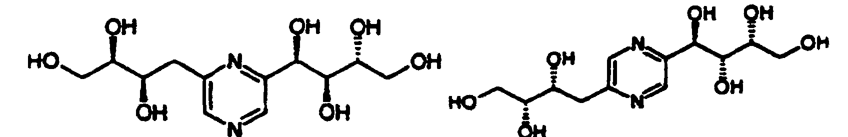

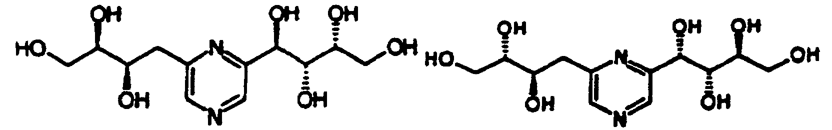

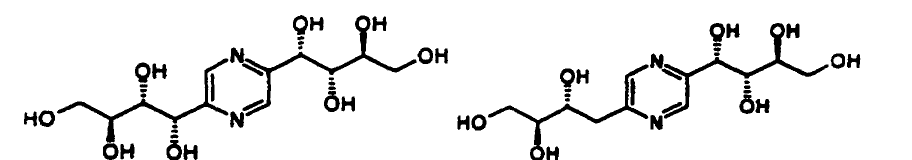

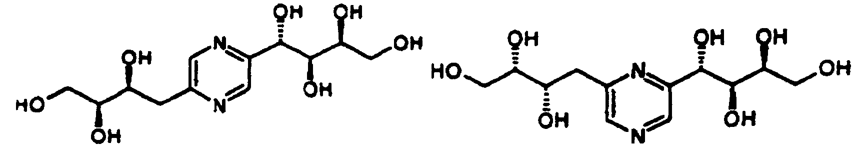

4. Following compounds of formula (I) according to claim 3:
1-[5-(1R,2R,3S,4-tetrahydroxybutyl)pyrazin-2-yl]butane-1R,2R,3S,4-tetraol
1-[5-(1S,2R,3R,4-tetrahydroxybutyl)pyrazin-2-yl]butane-1S,2R,3R,4-tetraol
1-[5-(1S,2S,3R,4-tetrahydroxybutyl)pyrazin-2-yl]butane-1S,2S,3R,4-tetraol
1-[5-(1S,2S,3S,4-tetrahydroxybutyl)pyrazin-2-yl]butane-1S,2S,3S,4-tetraol
1-[5-(2R,3S,4-trihydroxybutyl)pyrazin-2-yl]butane-1R,2R,3S,4-tetraol
1-[5-(2S,3S,4-trihydroxybutyl)pyrazin-2-yl]butane-1R,2S,3S,4-tetraol
1-[5-(2R,3R,4-trihydroxybutyl)pyrazin-2-yl]butane-1S,2R,3R,4-tetraol
1-[5-(2S,3R,4-trihydroxybutyl)pyrazin-2-yl]butane-1S,2S,3R,4-tetraol
1-[6-(2R,3S,4-trihydroxybutyl)pyrazin-2-yl]butane-1R,2R,3S,4-tetraol
1-[6-(2R,3R,4-trihydroxybutyl)pyrazin-2-yl]butane-1S,2R,3R,4-tetraol
1-[6-(2S,3R,4-trihydroxybutyl)pyrazin-2-yl]butane-1S,2S,3R,4-tetraol
or a salt of such a compound with an inorganic or organic acid.

5. Following compounds of formula (I) according to claim 3:
1-[5-(2S,3S,4-trihydroxybutyl)pyrazin-2-yl]butane-1R,2S,3S,4-tetraol
1-[5-(2R,3R,4-trihydroxybutyl)pyrazin-2-yl]butane-1S,2R,3R,4-tetraol
1-[5-(2S,3R,4-trihydroxybutyl)pyrazin-2-yl]butane-1S,2S,3R,4-tetraol
and their salts with an inorganic or organic acid.

6. Process for the preparation of the compounds of formula (I) according to claim 3 in which R₁ represents the stereoisomeric forms of the -(CHOH)₃-CH₂OH chain (II), R₂ represents a hydrogen atom and R₃ represents the stereoisomeric forms of the -CH₂-(CHOH)₂-CH₂OH chain (III), **characterized in that** ammonium formate is reacted with an aldose, or a mixture of 2 aldoses, of the dextrorotatory or laevorotatory series, of general formula:
CHO-CHOH-R₁ (X)
in which R₁ has the same meaning as in claim 3, the product is isolated and is optionally converted to a salt.

7. Process according to claim 6 in which the derivative of formula (X) is D-gulose, D-galactose, D-allose, D-altrose, D-idose, D-talose, L-glucose, L-mannose, L-galactose, L-allose, L-altrose, L-idose, L-talose or L-gulose.

8. Process for the preparation of the compounds of formula (I) according to claim 3 in which R₁ represents the stereoisomeric forms of the -(CHOH)₃-CH₂OH chain (II), R₂ represents the stereoisomeric forms of the -(CHOH)₃-CH₂OH chains (II) and R₃ represents a hydrogen atom, **characterized in that** an aminoaldose, or a mixture of 2 aminoaldoses, of general formula CHO-CH(NH₂)-R₁ (XI) in which R₁ has the same meaning as in claim 3, is treated in basic medium, the product is isolated and is optionally converted to a salt with an inorganic or organic acid.

9. Process according to claim 8 in which the aminoaldose of formula (XI) used is D-galactosamine.

10. Process for the preparation of the compounds of formula (I) according to claim 3 in which R₁ represents the stereoisomeric forms of the -(CHOH)₃-CH₂OH chain (II), R₂ represents the stereoisomeric forms of the -CH₂-(CHOH)₂-CH₂OH chains (III) and R₃ represents a hydrogen atom, **characterized in that** an aminoaldose, or a mixture of 2 aminoaldoses, of general formula CHO-CH(NH₂)-R₁ (XI) in which R₁ has the same meaning as in claim 3, is treated in acidic medium, the product is isolated and is optionally converted to a salt with an inorganic or organic acid.

11. Process according to claim 10 in which the derivative of formula (XI) is D-galactosamine.

12. Process for the preparation of the compounds of formula (I) according to claim 3 in which R₁ represents the stereoisomeric forms of the -(CHOH)₃-CH₂OH chain (II), R₂ represents the stereoisomeric forms of the -CH₂-(CHOH)₂-CH₂OH chains (III) and R₃ represents a hydrogen atom, **characterized in that** a ketose, or a mixture of 2 ketoses, of general formula HOCH₂-CO-R₁ (XII) in which R₁ has the same meaning as in claim 3, and ammonium formate are reacted, the product is isolated and is optionally converted to a salt with an inorganic or organic acid.

13. Process according to claim 12 in which the derivative of formula (XII) is D-psicose, D-sorbose, D-tagatose, L-psicose, L-fructose, L-sorbose or L-tagatose.

14. Use of the compounds of general formula (I). 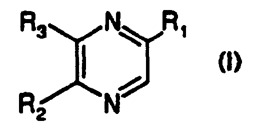 in which
R₁ represents the stereoisomeric forms of the chain
-(CHOH)₃-CH₂OH (II)
and
either R₂ represents a hydrogen atom and R₃ represents the stereoisomeric forms of the chain
-CH₂-(CHOH)₂-CH₂OH (III)
or R₂ represents the stereoisomeric forms of the chains
- (CHOH)₃-CH₂OH (II)
or
-CH₂-(CHOH)₂-CH₂OH (III)
and R₃ represents a hydrogen atom,
or their salts with an inorganic or organic acid, with the exception of
- fructosazine of formula 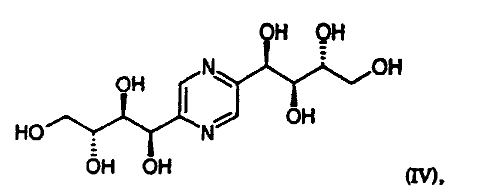
- deoxyfructosazine of formula 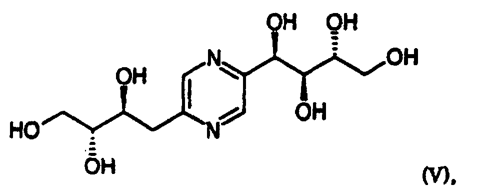
- and the compound of formula 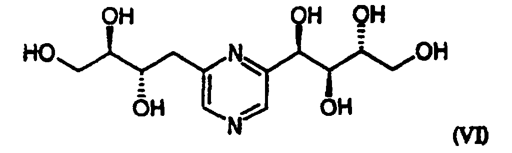 in the preparation of pharmaceutical compositions of use in the treatment or prevention of diabetes and complications of diabetes.
